(19) Europäisches Patentamt / European Patent Office / Office européen des brevets

(11) **EP 4 413 873 A1**

(12) **EUROPEAN PATENT APPLICATION**
published in accordance with Art. 153(4) EPC

(43) Date of publication:
**14.08.2024 Bulletin 2024/33**

(21) Application number: **22878617.4**

(22) Date of filing: **07.10.2022**

(51) International Patent Classification (IPC):
**A24B 15/167** $^{(2020.01)}$    **A24F 40/30** $^{(2020.01)}$

(52) Cooperative Patent Classification (CPC):
**A24B 15/167; A24F 40/30**

(86) International application number:
**PCT/JP2022/037597**

(87) International publication number:
**WO 2023/058750 (13.04.2023 Gazette 2023/15)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.10.2021 JP 2021166177**

(71) Applicant: **Japan Tobacco Inc.**
**Tokyo 105-6927 (JP)**

(72) Inventors:
• **MATSUMOTO, Hirofumi**
  **Tokyo 130-8603 (JP)**
• **NAKANO, Takuma**
  **Tokyo 130-8603 (JP)**
• **FUJIKURA, Hirofumi**
  **Tokyo 130-8603 (JP)**
• **SUGIURA, Mai**
  **Tokyo 130-8603 (JP)**

(74) Representative: **Hoffmann Eitle**
**Patent- und Rechtsanwälte PartmbB**
**Arabellastraße 30**
**81925 München (DE)**

(54) **LIQUID FOR ATOMIZATION AND PRODUCTION METHOD FOR SAME**

(57)    According to the present invention, a liquid for atomization includes organic acid ions A, metal ions M, and a medium. The liquid for atomization is to be contained in an atomization device without coming into contact with metal.

## Fig. 1

EP 4 413 873 A1

**Description**

TECHNICAL FIELD

The present invention relates to a liquid for atomization and a method for producing the same.

BACKGROUND ART

Non-combustible flavored smoking articles are known which generate a vapor or aerosol from an aerosol source material using electric power supplied by a battery, and pass the vapor or aerosol through a tobacco-containing raw material to add tobacco components to the vapor or aerosol (see, for example, Patent Literature 1). In order to achieve efficient delivery of nicotine, Patent Literature 2 discloses a liquid formulation comprising an aqueous solution of nicotine, an organic and/or inorganic salt, an organic liquid having a viscosity higher than water, and a particular amount of an organic alcohol, wherein the pH of the formulation is greater than 7 and the formulation does not include a propellant.

CITATION LIST

PATENT LITERATURE

> PTL 1: International Publication No. WO 2016/075749
> PTL 2: Japanese Unexamined Patent Application Publication (Translation of PCT Application) No. 2018-536014

SUMMARY OF INVENTION

TECHNICAL PROBLEM

A liquid for atomization is stored and transported in liquid form. There is, therefore, a fear of a hygiene problem that may occur over time due to bacteria or the like. A conceivable countermeasure is to add an acid to a liquid for atomization. However, the inventors found that mere addition of an acid, because of its corrosive action, may impair the stability of an article, or may corrode other part(s) upon leakage of the liquid. In view of such a situation, the present invention is directed to the provision of a liquid for atomization which improves the safety of a smoking article.

SOLUTION TO PROBLEM

The inventors found that a liquid containing an organic acid ion and a metal ion can solve the above problem.

Embodiment 1

A liquid for atomization, comprising an organic acid ion A, a metal ion M, and a medium, the liquid being to be stored in an atomizing device without contact with a metal.

Embodiment 2

A liquid for atomization, comprising an organic acid ion A, a metal ion M, and a medium, and containing no nicotine.

Embodiment 3

The liquid for atomization according to embodiment 1 or 2, wherein the metal of the metal ion M is selected from Group 1 or Group 2 of the periodic table.

Embodiment 4

The liquid for atomization according to any one of embodiments 1 to 3, wherein the organic acid ion A and the metal ion M are derived from an organic acid metal salt AM.

Embodiment 5

The liquid for atomization according to any one of embodiments 1 to 4, comprising a combination of the organic acid ion A and the metal ion M which satisfies the following relationship:

$$\text{(number of moles of organic acid ion A/number of moles of metal ion M)} \geq \text{(valence of A/valence of M)}.$$

Embodiment 6

The liquid for atomization according to any one of embodiments 1 to 5, having a pH of 4 to 8.5 when diluted 10-fold with water.

Embodiment 7

A non-combustible flavor inhalation article comprising: a reservoir holding the liquid according to any one of embodiments 1 to 6; and an atomizing device for atomizing the liquid supplied from the reservoir.

Embodiment 8

The non-combustible flavor inhalation article according to embodiment 7, further comprising a material holder holding a flavor source, wherein the atomized liquid is introduced into the material holder.

Embodiment 9

A method for producing the liquid for atomization according to any one of embodiments 1 to 6, the method comprising adding to the medium an organic acid metal salt, or an organic acid metal salt and an organic acid which produce a buffering effect.

ADVANTAGEOUS EFFECTS OF INVENTION

The present invention makes it possible to provide a liquid for atomization which reduces discomfort during smoking while ensuring the safety of a smoking article.

BRIEF DESCRIPTION OF DRAWINGS

[FIG. 1] FIG. 1 is a diagram showing an embodiment of a non-combustible flavor inhalation article.
[FIG. 2] FIG. 2 is a diagram showing an embodiment of a flavor source capsule.
[FIG. 3] FIG. 3 is a diagram showing an embodiment of a power supply unit.
[FIG. 4] FIG. 4 is a cross-sectional view of an embodiment of a cartridge.
[FIG. 5] FIG. 5 is a diagram showing the internal structure of the cartridge.
[FIG. 6] FIG. 6 is a diagram showing the amounts of ions in a liquid for atomization.

DESCRIPTION OF EMBODIMENTS

The present invention will now be descried in detail. In the present invention, "X to Y" includes the end values X and Y.

1. Liquid for Atomization

The liquid for atomization refers to a liquid which, when heated or vibrated, generates an aerosol. The liquid for atomization comprises an organic acid ion A, a metal ion M, and a medium. The inventors have found that while a liquid for atomization comprising an organic acid improves its hygiene reliability, it impairs the stability of an article due to the corrosiveness of the liquid, and have also found that the co-presence of an organic acid ion A and a metal ion M in a liquid can achieve both the hygiene reliability of the liquid and the stability of an article.

(1) Organic Acid Ion A, Metal Ion M

The metal of the metal ion M is preferably selected from Group 1 or Group 2 of the periodic table, and is preferably K, Na, Ca, or Mg from the viewpoint of availability, etc. The organic acid ion A is preferably derived from an aromatic organic acid such as benzoic acid, or a hydroxy fatty acid such as lactic acid, tartaric acid, citric acid, or levulinic acid. In particular, when nicotine is added to the liquid or when nicotine is contained in the below-described flavor source, the presence of the organic acid ion A can reduce smoke taste inhibition (a feeling of loss of flavor) caused by nicotine. This effect is pronounced when the organic acid ion A is derived from the above-described acid. Further, the use of a salt of the organic acid ion A makes it possible to prepare a liquid for atomization in which the minimum required amounts of the organic acid ion A and the metal ion M are dissolved in the medium. The above-described acid also has the advantage that the acid itself is unlikely to cause smoke taste inhibition.

The liquid for atomization may contain a plurality of types of organic acid ions and a plurality of types of metal ions. It is preferred that at least one pair be derived from an organic acid metal salt AM. Examples of the organic acid metal salt AM include sodium benzoate, potassium benzoate, calcium benzoate trihydrate, sodium lactate, trisodium citrate, or calcium lactate pentahydrate. For example, when $PhCOO^-$ (benzoate ion) exists as the organic acid ion A and $Na^+$ as the metal ion M in the liquid for atomization, these ions are preferably derived from sodium benzoate (PhCOONa).

In particular, the liquid for atomization preferably comprises at least one combination of the organic acid ion A and the metal ion M which satisfies the following inequality (1).

$$(\text{number of moles of organic acid ion A/number of moles of metal ion M}) \geq (\text{valence of A/valence of M}) \qquad (1)$$

For example, when the liquid for atomization contains 2 moles of sodium benzoate, the left side and the right side are as follows, satisfying inequality (1).

$$\text{Left side} = 2 \text{ moles (PhCOO-)} / 2 \text{ moles (Na+)} = 1$$

$$\text{Right side} = 1/1 = 1$$

When the liquid for atomization contains 2 moles of calcium tartrate, the left side and the right side are as follows, satisfying inequality (1).

$$\text{Left side} = 2 \text{ moles (tartrate ion}^{2-}） / 1 \text{ mole (Ca}^{2+}) = 2$$

$$\text{Right side} = 2/2 = 1$$

The liquid for atomization may contain an organic acid metal salt and an organic acid which produce a buffering effect. Also in this case, inequality (1) is preferably satisfied. For example, when the liquid for atomization contains 1 mole of sodium benzoate and 10 mols of benzoic acid as a combination which produces a buffering effect, the left side and the right side are as follows, satisfying inequality (1).

$$\text{Left side} = 11 \text{ moles (PhCOO}^-) / 1 \text{ mole (Na}^+) = 11$$

$$\text{Right side} = 1/1 = 1$$

The amounts of the organic acid ion A and the metal ion M are preferably selected so that the pH of the liquid for atomization falls within a particular range. In the present invention, the pH of the liquid for atomization is defined as the pH determined when the liquid is diluted 10-fold with water, and its value is preferably 4 to 8.5, more preferably 4 to 7.

(2) Medium

The liquid for atomization contains a liquid medium. The medium is preferably water or an aqueous organic solvent such as a polyhydric alcohol. Among them, a medium which is used as an aerosol source material in the art is more preferred. Thus, in one embodiment, the medium comprises an aerosol source material, such as a polyhydric alcohol, as a main component, and water preferably in an amount of 1 to 20% by weight, more preferably 3 to 10% by weight based on the amount of the aerosol source material.

### (3) Other Components

The liquid for atomization may or may not contain nicotine. When the liquid for atomization does not contain nicotine, it is possible to reduce a change in smoke taste, e.g. due to oxidation of a component contained in the liquid, thereby improving the quality stability. When the liquid for atomization contains nicotine, its amount is about 0.5 to 10% by weight based on the amount of the medium. When the liquid for atomization contains nicotine, the liquid achieves the effect of reducing smoke taste inhibition, as described above.

The organic acid ion A and the metal ion M have functions such as prevention of oxidation and prevention of deterioration. Therefore, when the liquid for atomization does not contain nicotine, the liquid can achieve the effect of reducing deterioration of the quality of other component(s), such as a flavoring agent, which may be contained in the liquid. When the liquid for atomization does not contain nicotine, the liquid is preferably used together with a tobacco raw material. Even when the organic acid and nicotine are stored and atomized separately, it is possible to reduce the smoke taste inhibition of an article as described above.

The liquid for atomization may contain other known components such as a flavoring agent.

### (4) Storage in Atomizing Device

The liquid for atomization is stored in an atomizing device. In one embodiment, the liquid for atomization is stored without contact with a metal. This can reduce problems that may occur e.g. upon leakage of the liquid for atomization, thereby further improving the safety of an article. In another embodiment, the liquid for atomization may be in contact with a metal when the liquid is stored in the atomizing device. The liquid for atomization according to this embodiment has low corrosiveness, and therefore can achieve high safety even when it is stored in contact with a metal.

### 2. Method for Producing Liquid for Atomization

While the liquid for atomization can be produced by any method, it is preferably produced by a method which includes (1) a step of adding an organic acid metal salt to a medium, or (2) a step of adding an organic acid metal salt and an organic acid, which produce a buffering effect, to a medium.

### (1) Step of Adding Organic Acid Metal Salt

The same organic acid metal salts as described above can be used as the organic acid metal salt. Such organic acid metal salts may be used singly or in a combination of two or more. The organic acid metal salt is preferably used in such an amount as to achieve the above-described pH.

### (2) Step of Adding an Organic Acid Metal Salt and an Organic Acid Which Produce a Buffering Effect

An example of the organic acid metal salt and the organic acid which produce a buffering effect is a combination of an acid and its salt whose organic ion species are the same. Specific examples include a combination of benzoic acid and a benzoate salt and a combination of lactic acid and a lactate salt. The total amount of the acid and the salt is preferably adjusted so that the above-described pH can be achieved. The weight ratio between the acid and the salt is preferably 1: (10-50). When the ratio is within this range, the large content of the salt can favorably increase the amount of the solute in the liquid for atomization and reduce the moisture activity of the liquid for atomization, and therefore can further reduce the occurrence of hygiene problems.

### 2. Non-Combustible Flavor Inhalation Article

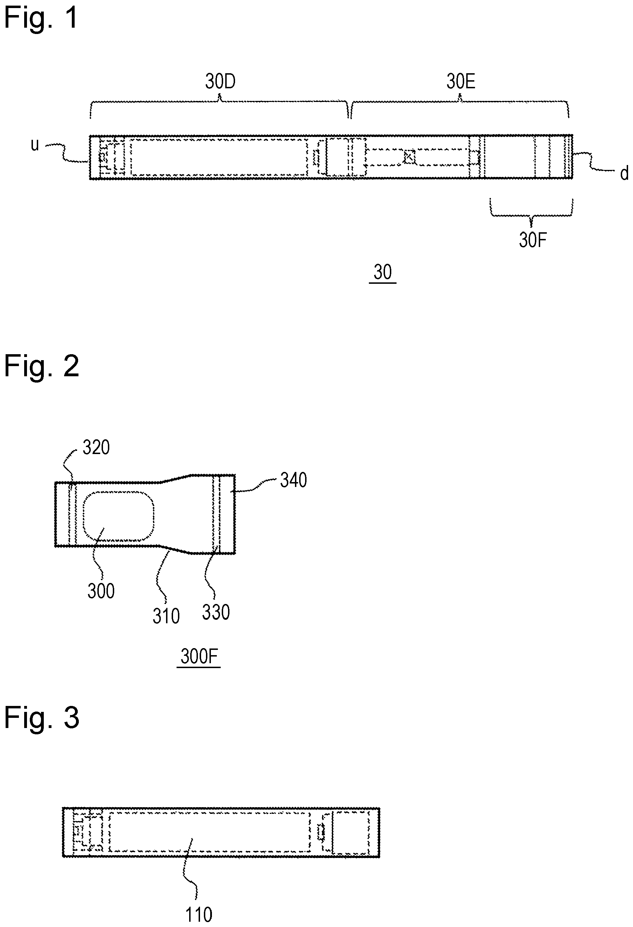

FIG. 1 shows an embodiment of a non-combustible flavor inhalation article. The non-combustible flavor inhalation article 30 includes a power supply unit 30D, a cartridge 30E, and a flavor source capsule 30F which is a material holder holding a flavor material. The non-combustible flavor inhalation article 30 has a shape extending from a non-inhalation end u (upstream) to an inhalation end d (downstream). The cartridge 30E is attachable/detachable to/from the power supply unit 30D. The flavor source capsule 30F is attachable/detachable to/from the cartridge 30E. The non-combustible flavor inhalation article of this embodiment is configured to generate an atomized liquid (aerosol) upstream of the material holder (flavor source capsule 30F), introduce the aerosol into the material holder, and cause the aerosol to carry flavor components to generate a flavor. In the case where the liquid for atomization contains nicotine, it is possible not to provide a material holder to the non-combustible flavor inhalation article.

1) Material Holder

In one embodiment, the material holder is the flavor source capsule 30F. As shown in FIG. 2, the flavor source capsule 30F includes a container 310 for storing the flavor source 300, a mesh body 320, a non-woven fabric 330, and a cap 340. The aerosol that has been atomized by the below-described atomizing section 220 is introduced through the mesh body 320 into the container 310, where the aerosol comes into contact with the flavor source 300, whereby a flavor is imparted to the aerosol. The aerosol then passes through the non-woven fabric 330 and is inhaled by the user. In this manner, the non-combustible flavor inhalation article 30 can impart a flavor to the aerosol without heating the flavor source 300. However, the flavor source 300 may be heated. In that case, the heating temperature is about 40 to 120°C. Substantially no aerosol is generated from the flavor source 300.

In the flow direction of the aerosol, the length of the flavor source capsule 30F (container 310) is preferably 40 mm or less, more preferably 25 mm or less. Further, in the flow direction of the aerosol, the length is preferably 1 mm or more, more preferably 5 mm or more. In a direction perpendicular to the flow direction of the aerosol, the maximum length of the container 310 of the flavor source capsule 30F (container 310) is preferably 20 mm or less, more preferably 10 mm or less. Further, in a direction perpendicular to the flow direction of the aerosol, the maximum length of the flavor source capsule 30F (container 310) is preferably 1 mm or more, more preferably 3 mm or more.

In one embodiment, the flavor source 300 is composed of pieces of tobacco raw material which impart a flavor to the aerosol. The lower limit of the size of each raw material piece is preferably 0.2 to 1.2 mm, more preferably 0.2 to 0.7 mm. The smaller the size of the raw material pieces, constituting the flavor source 300, the larger the specific surface area of the raw material pieces; therefore, inhaling flavor components are more easily released. Shredded tobacco or a shaped product obtained by shaping a tobacco raw material into granules, for example, can be used as the raw material pieces. The flavor source 300 may contain a plant(s) other than tobacco (e.g., mint or herb), a natural flagrance such as menthol, a synthetic flagrance, fruit juice, a taste agent, a plant powder, etc. Examples of the taste agent include materials which have sweetness, sourness, saltiness, umami, bitterness, astringency, richness, spiciness, harshness, etc. Examples of materials which have sweetness include a sugar, a sugar alcohol, and a sweetener. The sugar is, for example, a monosaccharide, a disaccharide, an oligosaccharide, or a polysaccharide. The sweetener is, for example, a natural sweetener or a synthetic sweetener.

The amount of the flavor source 300 charged into the container 310 is preferably 300 mg or more, more preferably 350 mg or more from the viewpoint of increasing the amount of nicotine volatilized during smoking.

When the flavor source 300 contains pieces of tobacco raw material, the liquid for atomization need not contain nicotine. On the other hand, when the flavor source 300 does not contain pieces of tobacco raw material or when the non-combustible flavor inhalation article 30 does not include a material holder, the liquid for atomization preferably contains nicotine.

2) Power supply unit

FIG. 3 shows an example of the power supply unit 30D. The power supply unit 30D includes a battery 110. The battery 110 may be a disposable battery or a rechargeable battery. The initial value of the output voltage of the battery 110 is preferably in the range of 1.2 V to 4.2 V. The battery capacity of the battery 110 is preferably in the range of 100 mAh to 1000 mAh.

3) Cartridge

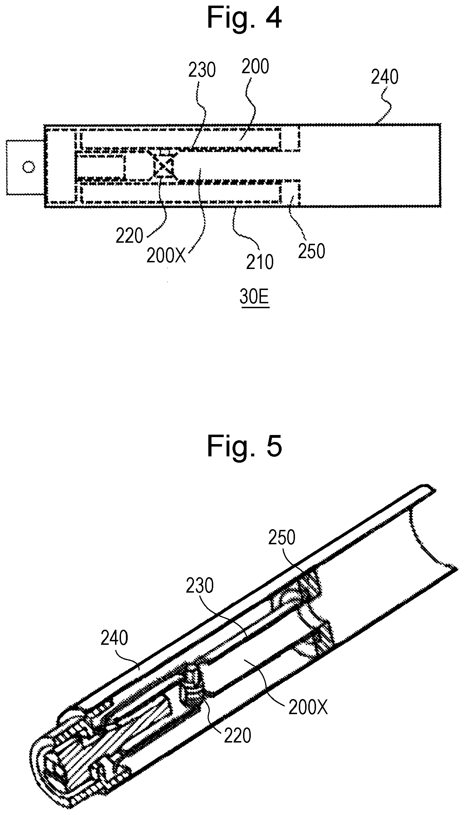

FIGS. 4 and 5 show an example of the cartridge 30E. FIG. 4 is a cross-sectional view of the example of the cartridge 30E, and FIG. 5 is a diagram showing its internal structure. The cartridge 30E includes a reservoir 210, an atomizing section 220, a flow path forming body 230, an outer frame 240, and an end cap 250. The cartridge 30E has a first flow path 200X as an aerosol flow path, which is disposed downstream of the atomizing section 220.

The reservoir 210 stores the liquid for atomization 200. The reservoir 210 is located around the flow path forming body 230 in a cross-section perpendicular to the aerosol flow direction (direction from the non-inhalation end toward the inhalation end (upstream to downstream)). The reservoir 210 is located in the space between the flow path forming body 230 and the outer frame 240. The reservoir 210 is formed of, for example, a non-metallic porous body such as a resin web or cotton. Alternatively, the reservoir 210 may be formed of a tank for storing the liquid for atomization 200. In that case, the tank is made of, for example, a polymer.

The atomizing section 220 atomizes, without involving combustion, the liquid for atomization 200 using electric power supplied from the battery 110. The atomizing section 220 is composed of a heating wire (coil) wound at a predetermined pitch. The atomizing section 220 is preferably composed of a heating wire having a resistance value in the range of 1.0 to 3.0 $\Omega$. The predetermined pitch is preferably at least a value at which adjacent heating wires do not contact each other, and a smaller possible value is preferred. For example, the predetermined pitch is preferably 0.40 mm or less. The predetermined pitch is preferably constant in order to stabilize the atomization of the liquid for atomization 200. The predetermined pitch refers to the distance between the centers of adjacent heating wires. Alternatively, the atomizing section 220 may be comprised of a ceramic heater.

The liquid for atomization 200, held in the reservoir 210, is stored in a state in which it is supplied to the atomizing section through capillarity or the like. When the atomizing section is composed of a heating wire, the liquid for atomization is in contact with a metal when it is stored in the non-combustible flavor inhalation article (atomizing device). However, since the liquid for atomization according to this embodiment has low corrosiveness, it can achieve high stability of the device even when it is stored in such a state. In the case where the atomizing section is comprised of a ceramic heater, the liquid for atomization may or may not be in contact with a metal when it is stored in the non-combustible flavor inhalation article (atomizing device). Since the liquid for atomization has low corrosiveness to a metal component(s) of the ceramic heater, it can achieve high stability of the device.

The flow path forming body 230 has a cylindrical shape that forms the first flow path 200X extending along the flow direction of the aerosol. The outer frame 240 has a cylindrical shape that houses the flow path forming body 230. The outer frame 240 extends downstream from the end cap 250, while it houses a portion of the flavor source capsule 30F. The end cap 250 is a cap that closes the space between the flow path forming body 230 and the outer frame 240 from the downstream side. The end cap 250 prevents the liquid for atomization 200, stored in the reservoir 210, from leaking to the flavor source capsule 30F.

The liquid for atomization according to this embodiment is also suitable for a flavor inhaler of a type which atomizes the liquid by a method other than the method as illustrated in the figures. Such methods include, for example, a method which involves applying ultrasonic vibrations to the liquid to atomize it, a method which uses an element that deforms when energized, and a method which uses bubbles generated in the liquid.

The method which uses an element is, for example, a surface acoustic wave (SAW) method or a piezo method. The surface acoustic wave (SAW) method is a method which involves applying a voltage at a high frequency to a piezoelectric substrate, having a pair of comb-shaped electrodes, to generate surface acoustic waves (SAW), and atomizing a liquid by means of the surface acoustic waves, as disclosed in International Publication No. WO 2021/039340, the contents of which are incorporated herein by reference.

The piezo method is a method which atomizes a liquid by vibrating it using a piezoelectric element (piezo element) which deforms when energized.

The method which uses bubbles is, for example, a thermal method. The thermal method is a method which involves boiling a liquid instantaneously in a nozzle equipped with an internal heater to generate bubbles, and ejecting and atomizing the liquid by means of the bubbles. The thermal method and the piezo method are also known as a drop-on-demand method in the field of inkjet printing.

EXAMPLES

[Comparative Example 1]

Propylene glycol, glycerin, and water were provided as a medium, and benzoic acid (BA) was provided as an organic acid. 5% by weight of water was added to a mixture of propylene glycol and glycerin, which had been mixed at a weight ratio of 1:1, and the organic acid was added in the amounts shown in Table 1 to the medium to prepare liquids 1 and 2 for atomization. Next, a non-combustible flavored smoking article as shown in FIG. 1 was provided, and each liquid for atomization was filled into the liquid holder of the article. For the non-combustible flavored smoking article, a smoking test was conducted by well-trained panelists to evaluate smoke taste inhibition.

[Example 1]

Propylene glycol, glycerin, and water were provided as a medium, sodium benzoate (BA-Na) was provided as an organic acid salt, and benzoic acid (BA) was provided as an organic acid. 5% by weight of water was added to a mixture of propylene glycol and glycerin, which had been mixed at a weight ratio of 1:1, and the organic acid salt and the organic acid were added in the amounts shown in Table 1 to the medium to prepare liquids 3 to 5 for atomization. Next, for the non-combustible flavored smoking article, a smoking test was conducted by well-trained panelists in the same manner as in Comparative Example 1 to evaluate the effect of reducing smoke taste inhibition (A: very high reduction effect, B: high reduction effect, C: no reduction effect). The results are shown in Table 1. Table 1 also shows the pH of each liquid as measured when the liquid was diluted 10-fold with water.

[Table 1]

| | Liquid No. | medium | | wt % | | mol/L | | | pH | Smoke taste inhibition |
|---|---|---|---|---|---|---|---|---|---|---|
| | | PG:G | Water wt % | BA | BA-Na | BA | BA-Na | Total | | |
| Comp. Example 1 | 1 | 1:1 | 5 | - | - | - | - | - | - | C |
| | 2 | 1:1 | 5 | 2.5 | - | 0.23 | - | 0.23 | 3.0 | A |
| Example 1 | 3 | 1:1 | 5 | - | 3.0 | - | 0.22 | 0.22 | 8.3 | B |
| | 4 | 1:1 | 5 | 0.1 | 2.9 | 0.01 | 0.21 | 0.22 | 5.6 | B |
| | 5 | 1:1 | 5 | 0.2 | 6.0 | 0.02 | 0.46 | 0.48 | 5.6 | B |

[Example 2]

The liquids 3 to 5 for atomization were provided, and each liquid was filled into the liquid holder of the non-combustible flavored smoking article used in Example 1. The article was stored at 40°C for 3 months. After storage, the liquid for atomization was taken out from the article, and the amounts of metal ions were measured by ion chromatography.

[Comparative example 2]

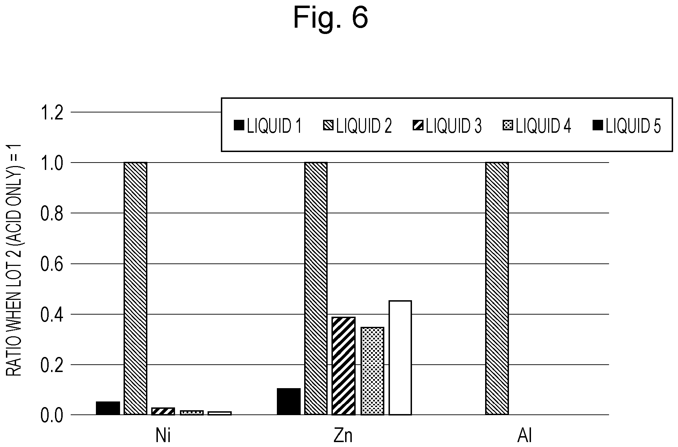

The amounts of metal ions were measured in the same manner as in Example 2 except that the liquids 1 and 2 for atomization were used instead of the liquids 3 to 5 for atomization. The results are shown in FIG. 6. In the figure, the measured amounts of metal ions in the liquid 2 for atomization are indicated as 1. The amounts of metal ions were small in all of the liquids for atomization obtained in the Examples; in particular, no Al was detected.

The results clearly indicate that the liquids for atomization according to this embodiment are less corrosive to other materials and have higher safety. The results also indicate that the liquids for atomization according to this embodiment reduce flavor inhibition during smoking.

REFERENCE SIGNS LIST

| | |
|---|---|
| 30 | non-combustible flavor inhalation article |
| 30D | power supply unit |
| 30E | cartridge |
| 30F | flavor source capsule |
| u | non-inhalation end |
| d | inhalation end |
| 110 | battery |
| 200 | liquid for atomization |
| 210 | reservoir |
| 220 | atomizing section |
| 230 | flow path forming body |
| 240 | outer frame |
| 250 | end cap |
| 200X | first flow path |
| 300 | flavor source |
| 310 | container |
| 320 | mesh body |
| 330 | non-woven fabric |
| 340 | cap |

**Claims**

1. A liquid for atomization, comprising an organic acid ion A, a metal ion M, and a medium, the liquid being to be stored in an atomizing device without contact with a metal.

2. A liquid for atomization, comprising an organic acid ion A, a metal ion M, and a medium, and containing no nicotine.

3. The liquid for atomization according to claim 1 or 2, wherein the metal of the metal ion M is selected from Group 1 or Group 2 of the periodic table.

4. The liquid for atomization according to any one of claims 1 to 3, wherein the organic acid ion A and the metal ion M are derived from an organic acid metal salt AM.

5. The liquid for atomization according to any one of claims 1 to 4, comprising a combination of the organic acid ion A and the metal ion M which satisfies the following relationship:

$$\text{(number of moles of organic acid ion A/number of moles of metal ion M)} \geq \text{(valence of A/valence of M)}.$$

6. The liquid for atomization according to any one of claims 1 to 5, having a pH of 4 to 8.5 when diluted 10-fold with water.

7. A non-combustible flavor inhalation article comprising: a reservoir holding the liquid according to any one of claims 1 to 6; and an atomizing device for atomizing the liquid supplied from the reservoir.

8. The non-combustible flavor inhalation article according to claim 7, further comprising a material holder holding a flavor source, wherein the atomized liquid is introduced into the material holder.

9. A method for producing the liquid for atomization according to any one of claims 1 to 6, the method comprising adding to the medium an organic acid metal salt, or an organic acid metal salt and an organic acid which produce a buffering effect.

Fig. 1

Fig. 2

Fig. 3

# Fig. 4

30E

# Fig. 5

# Fig. 6

## INTERNATIONAL SEARCH REPORT

| International application No. |
|---|
| **PCT/JP2022/037597** |

### A. CLASSIFICATION OF SUBJECT MATTER

*A24B 15/167*(2020.01)i; *A24F 40/30*(2020.01)i
FI:  A24B15/167; A24F40/30

According to International Patent Classification (IPC) or to both national classification and IPC

### B. FIELDS SEARCHED

Minimum documentation searched (classification system followed by classification symbols)

A24B15/167; A24F40/30

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2020-504999 A (PHILIP MORRIS PRODUCTS S.A.) 20 February 2020 (2020-02-20) paragraphs [0004], [0035]-[0039], [0061]-[0067], [0071]-[0075], [0080]-[0088], fig. 1-4 | 2-5, 7, 9 |
| Y | | 1, 6, 8 |
| Y | JP 2002-119328 A (HOYU CO LTD) 23 April 2002 (2002-04-23) paragraph [0005] | 1, 6, 8 |
| Y | WO 2021/201265 A1 (JAPAN TOBACCO INC.) 07 October 2021 (2021-10-07) paragraphs [0015]-[0024], fig. 1-5 | 1, 6, 8 |
| Y | US 2021/0169121 A1 (NICOVENTURES TRADING LIMITED) 10 June 2021 (2021-06-10) paragraphs [0072]-[0081] | 6, 8 |

☐ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **17 November 2022** | **29 November 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/037597**

| Patent document cited in search report | | | Publication date (day/month/year) | Patent family member(s) | | | Publication date (day/month/year) |
|---|---|---|---|---|---|---|---|
| JP | 2020-504999 | A | 20 February 2020 | US 2018/0220697 A1 paragraphs [0004], [0038]-[0042], [0063]-[0070], [0074]-[0078], [0083]-[0091], fig. 1-4 WO 2018/141941 A2 EP 3576552 A2 CN 110167365 A KR 10-2019-0114964 A | | | |
| JP | 2002-119328 | A | 23 April 2002 | (Family: none) | | | |
| WO | 2021/201265 | A1 | 07 October 2021 | (Family: none) | | | |
| US | 2021/0169121 | A1 | 10 June 2021 | WO 2021/116890 A1 | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016075749 A
- JP 2018536014 W

- WO 2021039340 A